(19) Europäisches Patentamt

European Patent Office

Office européen des brevets

(11) **EP 1 078 603 B1**

(12) **EUROPÄISCHE PATENTSCHRIFT**

(45) Veröffentlichungstag und Bekanntmachung des Hinweises auf die Patenterteilung:
**15.12.2004 Patentblatt 2004/51**

(51) Int Cl.⁷: **A61B 17/132**, A44B 11/26, B65D 63/10

(21) Anmeldenummer: **99116381.7**

(22) Anmeldetag: **20.08.1999**

(54) **Venenstauvorrichtung**

Vein occluding device

Dispositif d'occlusion veineuse

(84) Benannte Vertragsstaaten:
**AT BE CH CY DE DK ES FI FR GB GR IE IT LI LU MC NL PT SE**

(43) Veröffentlichungstag der Anmeldung:
**28.02.2001 Patentblatt 2001/09**

(73) Patentinhaber: **PRÄMETA GmbH & Co. KG**
**51107 Köln (DE)**

(72) Erfinder:
• **Wehking, Wolfgang**
**51145 Köln (DE)**

• **Wasilewski, Wladyslaw Dipl. Ing.**
**51105 Köln (DE)**

(74) Vertreter: **Dallmeyer, Georg, Dipl.-Ing. et al**
**Patentanwälte**
**Von Kreisler-Selting-Werner**
**Bahnhofsvorplatz 1 (Deichmannhaus)**
**50667 Köln (DE)**

(56) Entgegenhaltungen:
**EP-A- 0 513 485**      **DE-A- 3 624 112**
**GB-A- 2 138 490**

**Beschreibung**

Die Erfindung bezieht sich auf eine Venenstauvorrichtung für Körperteile, mit einem eine Schlaufe bildenden Band, mit einem Schloßgehäuse und einem in dem Schloßgehäuse schwenkbar angeordneten Klemmhebel zum Klemmen des durch das Schloßgehäuse hindurchgeführten Bandabschnittes mit einem ersten Ende des Klemmhebels bei gespannter Schlaufe, wobei ein Ende des Bandes an dem zweiten Ende des Klemmhebels angreift.

Die bekannten Venenstauvorrichtungen bzw. Abschnürrvorrichtung für Körperteile sind üblicherweise so ausgebildet, daß zur schwenkbaren Lagerung des Klemmhebels ein Achsstift vorgesehen ist, der beiderseits in den Seitenwangen des Schloßgehäuses gelagert ist. Der Klemmhebel ist daher fest in dem Schloßgehäuse integriert. Das eine Ende des Bandes ist durch das Schloßgehäuse hindurchgeführt und wird in diesem bei um das Körperteil gespannter Schlaufe mittels des Klemmhebels in seiner Position innerhalb des Schloßgehäuses festgehalten. Das andere Ende des Bandes ist mittels eines Halteteils an dem der Klemmstelle gegenüberliegenden Ende des Klemmhebels einrastbar. Bei Druckausübung auf den Klemmhebel 1 kann das gespannte Band gelöst werden.

Wird Druck auf das Halteorgan ausgeübt, kann das Bandende von dem Klemmhebel gelöst werden.

Eine derartige Venenstauvorrichtung ist beispielsweise aus der EP-A-0 374 464 bekannt. Nachteilig an der bekannten Konstruktion einer Venenstauvorrichtung ist die Vielzahl der erforderlichen Einzelteile, sowie der Montageaufwand zur Herstellung der Venenstauvorrichtung. Eine weitere Venenstauvorrichtung ist beispielsweise aus DE 3624112 A1 bekannt. Der Vorteil dieser Venenstauvorrichtung soll sein, dass eine falsche Betätigung beim Entriegeln, die einen Stauschock auslösen kann, nicht möglich ist. Ferner ist der Klemmhebel der Venenstauvorrichtung einteilig mit einer Taste ausgebildet. Durch einen leichten Druck auf die Tast soll sich das Venenstauband langsam lösen. Bei einem deutlich stärkeren Druck auf die Taste soll es zu einer Entriegelung des Klemmhebels kommen. Nachteilig bei dieser Ausführungsform ist, dass eine Unterscheidung zwischen einem leichten Druck und einem deutlich stärkeren Druck schwierig ist, so dass es zu einem unerwünschten Entriegeln der Venenstauvorrichtung mit dem darausfolgendem Stauschock kommen kann.

Der Erfindung liegt die Aufgabe zugrunde, eine Venenstauvorrichtung der eingangs genannten Art derart zu verbessern, daß Herstellung und Montage vereinfacht werden und dabei gleichzeitig die Bedienungssicherheit verbessert wird.

Zur Lösung dieser Aufgabe dienen die Merkmale des Anspruchs 1.

Der Klemmhebel weist zwei Tastflächen auf, von denen eine erste unmittelbar auf das zweite Ende des Klemmhebels einwirkt und bei Betätigung den Klemmhebel verschwenkt. Eine zweite Tastfläche löst bei Betätigung die Entsperreinrichtung aus und gibt den Klemmhebel aus dem Schloßgehäuse frei. Bei dieser Lösung ist daher vorgesehen, dass der einrastbare Klemmhebel zwei Tastflächen für die Funktion "Entstauen" bzw. die Funktion "Lösen" aufweist.

Der Klemmhebel ist aus zwei an einem Ende miteinander verbundenen Schenkelteilen gebildet. Ein solches Teil kann als Spritzgußteil hergestellt werden und zumindest teilweise elastisch gestaltet werden, um eine elastische Rastverbindung bzw. Entsperreinrichtung zu bilden.

Die Erfindung sieht in vorteilhafter Weise vor, dass das Band mit dem unteren Schenkelteil des Klemmhebels verbunden ist und dass der Klemmhebel eine erste Tastfläche aufweist, die unmittelbar auf das zweite Ende des Klemmhebels einwirken und bei Betätigung den Klemmhebel ohne Entsperren verschwenken kann.

Der Klemmhebel einer solchen Venenstauvorrichtung ermöglicht eine Doppelfunktion. Allein mit dem Klemmhebel kann sowohl die Funktion "Entstauen" der Bandschlaufe als auch die Funktion "Lösen" der Bandschlaufe mit einem einzigen Teil verwirklicht werden. Dadurch wird der Aufwand für die Teileherstellung und Montage reduziert.

Vorzugsweise ist vorgesehen, dass der obere Schenkelteil elastisch schwenkbar ist und eine Entsperrvorrichtung bildet, die die zweite Tastfläche aufweist.

Vorzugsweise ist der Klemmhebel ein einstückiges Teil.

Der untere Schenkelteil kann als zweiarmiger Hebel eine nach unten abstehende, quer zum Band verlaufende Abstützung aufweisen, die die Schwenkachse des Klemmhebels bildet und die auf dem zwischen dem Klemmhebel und dem unteren Teil des Schloßgehäuses verlaufenden Band aufliegt. Die Schwenkachse muß nicht in dem Schloßgehäuse gelagert sein, da der Klemmhebel aufgrund der Rastverbindung, die eine gewisse Schwenkbewegung des Klemmhebels zuläßt, in seiner Position in dem Schloßgehäuse gehalten wird. Es ist daher lediglich erforderlich, die Schwenkachse in Form eines quer zum Band verlaufenden Steges auszubilden, wobei der vorstehende Steg vorzugsweise eine halbrunde Form und eine glatte Oberfläche aufweist.

Der obere Schenkelteil verrastet mit dem oberen Teil des Schloßgehäuses über die Rastverbindung und verriegelt damit den Klemmhebel im Schloßgehäuse.

Bei einem bevorzugten Ausführungsbeispiel ist die erste Tastfläche mit dem unteren Schenkelteil des Klemmhebels gekoppelt und wirkt bei Betätigung auf das mit dem Band verbundenen Ende des Klemmhebels ein, wodurch das gespannte Die zweite Tastfläche ist mit dem oberen Schenkelteil gekoppelt und bildet die Entsperreinrichtung, die bei Betätigung die Rastverbindung löst, wodurch der Klemmhebel aus dem Schloßgehäuse entnehmbar ist und die Schlaufe des Bandes geöffnet wird. Im einfachsten Fall ist die zweite Tastfläche einstückig mit dem oberen Schenkelteil verbunden, so daß der obere Schenkelteil bei Betätigung der zweiten Tastfläche elastisch verformt wird, bis die aus einem Vorsprung und aus einer Aussparung bestehende Rastverbindung entkoppelt ist. Dadurch ist der Klemmhebel aus dem Schloßgehäuse entnehmbar.

Die Rastverbindung besteht aus einem Vorsprung und einer dem Vorsprung angepaßten Aussparung. Der Vorsprung kann an dem oberen Schenkelteil des Klemmhebels ausgebildet sein und in eine Aussparung des Schloßgehäuses im oberen Teil des Schloßgehäuses eingreifen. Es ist aber auch möglich, daß der Vorsprung an dem oberen Teil des Schloßgehäuses nach innen vorsteht und in eine Aussparung des Klemmhebels eingreift.

Im folgenden werden unter Bezugnahme auf die Zeichnungen Ausführungsbeispiele der Erfindung näher erläutert:

Es zeigen:

Fig. 1        eine perspektivische Darstellung der Venenstauvorrichtung,

Fig. 2        das Funktionsprinzip des Klemmhebels,

Fig. 3        einen Längsschnitt durch die Venenstauvorrichtung, und

Fign. 4 bis 7        unterschiedliche Tastenanordnungen auf dem Klemmhebel.

Die Venenstauvorrichtung 1 als Abschnürvorrichtung für Körperteile weist ein Schloßgehäuse 4 auf, in dem ein Klemmhebel 6 um eine Achse schwenkbar angeordnet ist. Der Klemmhebel 6 dient zum Klemmen eines Bandabschnittes 3 eines eine Schlaufe bildenden Bandes 2. Ein Ende 8 des Bandes ist an dem hinteren Ende 10 des Klemmhebels 6 befestigt, wobei das andere Ende des Bandes durch das Schloßgehäuse hindurchgeführt ist. Ein Bandabschnitt 3 des Bandes 2 wird in dem Schloßgehäuse 4 durch den Klemmhebel 6 festgeklemmt, sobald die Schlaufe des Bandes 2 unter Spannung steht. Hierzu wird das Band 2 um das abzuschnürende Körperteil gelegt, wobei der Klemmhebel 6 vorher oder nachträglich in das Schloßgehäuse 4 eingeführt und dort mit Hilfe einer Rastverbindung 12 verrastet wird. Anschließend wird an dem freien Bandende gezogen bis die Schlaufe des Bandes 2 das Körperteil eng umfaßt und unter Spannung steht. Steht die Schlaufe 2 unter Spannung, wird der Klemmhebel 6 so verschwenkt, daß er mit dem vorderen Ende 7 eine Klemmstelle für den Bandabschnitt 3 in dem Schloßgehäuse 4 bildet, so daß eine Selbsthemmung des Bandes eintritt.

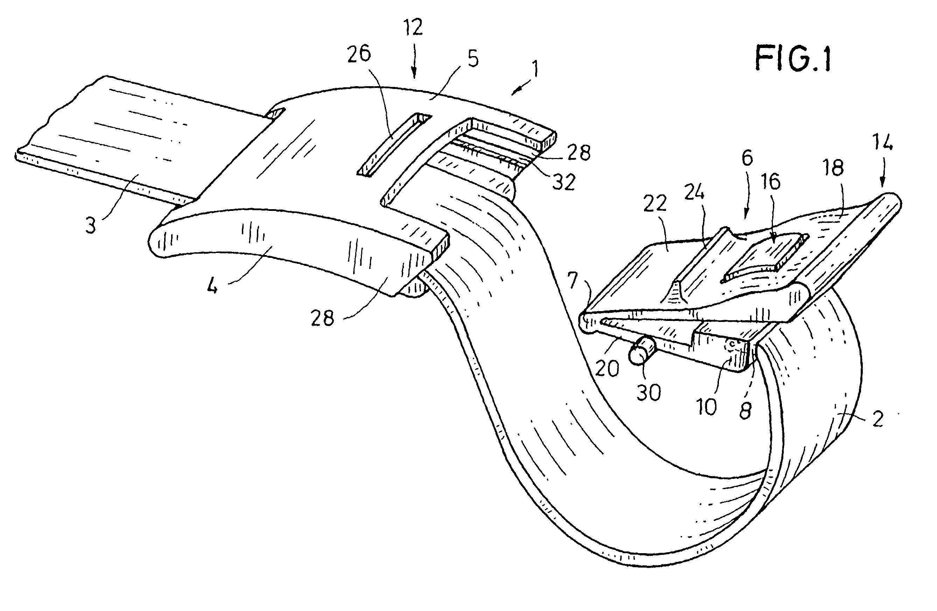

Der Klemmhebel 6 ist in dem Schloßgehäuse 4 einrastbar, wobei die Rastverbindung 12 aus einem Vorsprung 24 an dem oberen Schenkelteil 22 und einer Aussparung 26 in dem oberen Teil 5 des Schloßgehäuses 4 besteht. Mittels einer Entsperreinrichtung 14 ist der Klemmhebel 6 von dem Schloßgehäuse 4 lösbar. Bei dem in Fig. 1 gezeigten Ausführungsbeispiel besteht die Entsperreinrichtung 14 aus einer Tastfläche 18 am hinteren Ende des oberen Schenkelteils 22 des Klemmhebels 6. Bei Betätigung dieser Tastfläche 18 wird der Vorsprung 24 außer Eingriff mit der Aussparung 26 gebracht, so daß der Klemmhebel 6 vollständig aus dem Schloßgehäuse entnehmbar ist.

Der aus zwei an dem vorderen Ende 7 des Klemmhebels miteinander verbundenen Schenkelteilen 20,22 gebildete Klemmhebel 6 weist eine weitere Tastfläche 16 auf, die starr mit dem unteren Schenkelteil 20 verbunden ist und bei dem Ausführungsbeispiel der Fig. 1 durch den oberen Schenkelteil 22 hindurchtritt. Bei Betätigung der Tastfläche 16 kann der Klemmhebel 6 an seinem hinteren Ende 10 niedergedrückt werden, wodurch die Klemmstelle des Bandabschnittes 3 am vorderen Ende 7 des Klemmhebels 6 gelöst wird und die gespannte Schlaufe des Bandes 2 durch Freigeben des Bandabschnittes 3 entspannt werden kann.

Nach dem Entspannen der Bandschlaufe kann durch Betätigung der Tastfläche 18 der Klemmhebel 6 vollständig aus dem Schloßgehäuse entnommen werden. Hierzu ist der obere Schenkelteil 22 bei dem Ausführungsbeispiel der Fign. 1 bis 3 elastisch verschwenkbar.

Der Klemmhebel 6 kann als einstückiges Teil, z.B. als Spritzgußteil, hergestellt werden, wodurch sowohl Produktionskosten als auch Montagekosten reduziert werden.

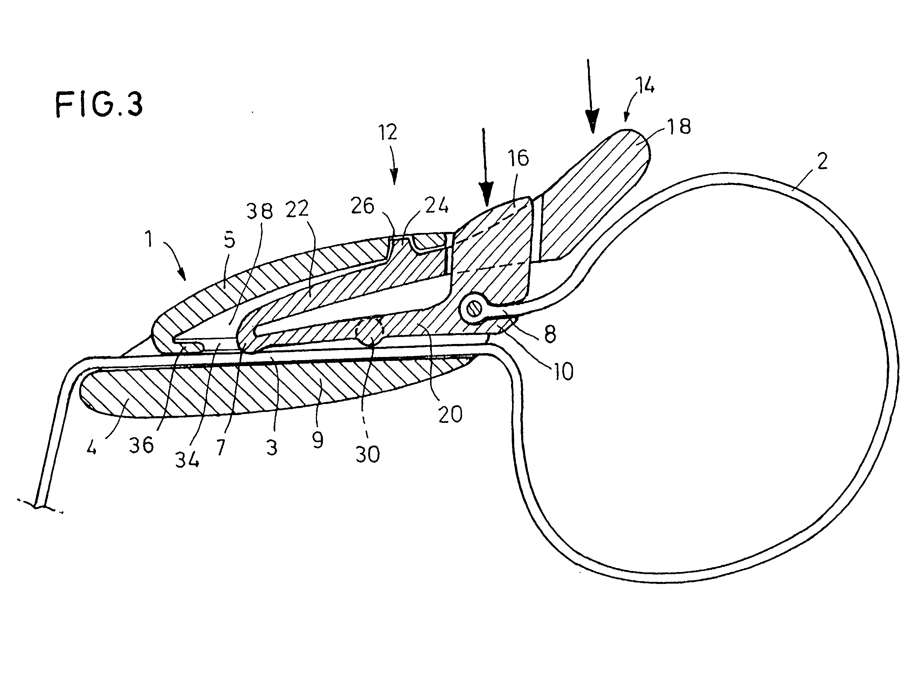

Der untere Schenkelteil 20 des Klemmhebels 6 ist als zweiarmiger Hebel gestaltet, der eine quer zum Band verlaufende Schwenkachse aufweist. Bei den Ausführungsbeispielen der Fign. 1 und 3 ist die Schwenkachse durch seitliche Zapfen 30 definiert, die in Nuten 32 der Seitenwände 28 des Schloßgehäuses einführbar sind. Dadurch ist die Lage der Schwenkachse in dem Schloßgehäuse 4 exakt definiert.

Es ist allerdings auch möglich, anstelle der Zapfen 30 und der Nuten 32 lediglich einen im Querschnitt halbrunden Steg auf der Unterseite des unteren Schenkelteils 20 vorzusehen, der auf dem Bandabschnitt 3 aufliegt und die Schwenkachse bildet. Dabei ist der Steg möglichst glatt und großflächig zu gestalten, damit der Bandabschnitt 3 unter diesem Steg gleiten kann.

Dagegen sollte die Unterseite des Klemmhebels 6 an dem vorderen Ende 7 reibungserhöhende Mittel aufweisen, beispielsweise eine spitz zulaufende, quer zum Band 2 verlaufende Kante.

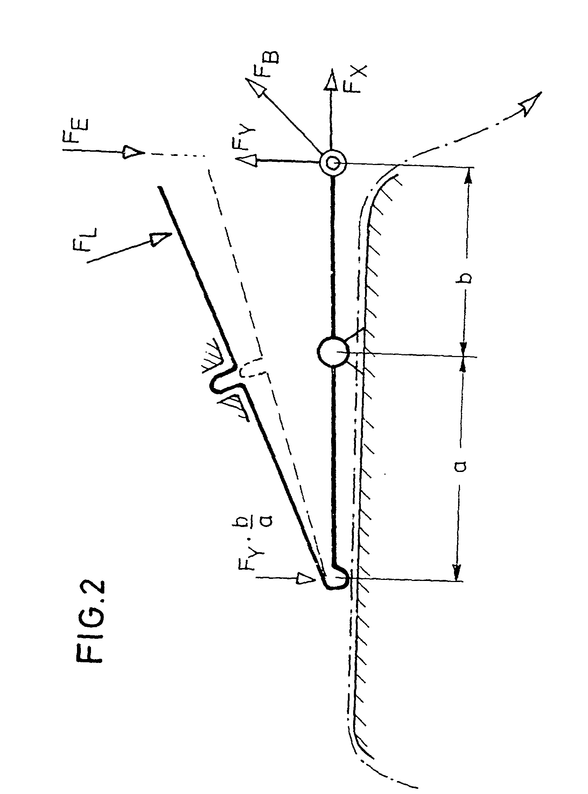

Fig. 2 zeigt eine schematische Darstellung der auf den Klemmhebel 6 einwirkenden Kräfte. Bei gespanntem Band wirkt die Kraft $F_B$ an dem hinteren Ende 10 des unteren Schenkelteils 20 in einer schräg nach oben weisenden Richtung. Die Kraft $F_B$ kann in zwei Komponenten aufgeteilt werden, nämlich in eine orthogonal zu dem unteren Schenkelteil verlaufenden Kraft $F_Y$ und einer in Richtung des unteren Schenkelteils 20 verlaufenden $F_X$. Die Kraft $F_Y$ führt an der Klemmstelle am vorderen Ende 7 des unteren Schenkelteils 20 des Klemmhebels 6 zu einer Klemmkraft auf dem Bandabschnitt 3, die sich aus $F_Y \times \frac{b}{a}$ ergibt. "a" und "b" geben die Hebelverhältnisse des als zweiarmigen Hebel gestalteten Schenkelteils 20 in bezug auf die Schwenkachse an. Zum Entspannen des Bandes muß daher über die Tastfläche 16 eine Kraft $F_E$ aufgebracht werden, die die Kraft $F_Y$ übersteigt, wodurch der Bandabschnitt 3 an der Klemmstelle am vorderen Ende 7 des Klemmhebels 6 freigegeben wird.

Zum vollständigen Lösen des Klemmhebels 6 aus dem Schloßgehäuse 4 muß eine Kraft $F_L$ auf den oberen Schenkelteil 22 des Klemmebels 6 aufgebracht werden, um den elastischen oberen Schenkelteil 22 zu verschwenken, so daß die Rastverbindung 12 außer Eingriff kommt. Das Schloßgehäuse 4 nimmt dabei über die Rastverbindung 12 die Bandzugkraftkomponente $F_X$ auf .

Fig. 3 zeigt einen Längsschnitt durch die Venenstauvorrichtung 1, wobei erkennbar ist, daß das Schloßgehäuse 4 einen Hohlraum 38 zur Aufnahme des Klemmhebels 6, sowie einen Durchlaß für das Band 2 aufweist. Der Bandabschnitt 3 ist dabei unterhalb des schwenkbar in dem Schloßgehäuse gelagerten Klemmhebels 6 geführt. Bei dem Ausführungsbeispiel mit seitlichen Zapfen 30 berühren diese den Bandabschnitt 3 nicht.

Das Schloßgehäuse 4 kann von den Seitenwänden 28 nach innen abstehende Bandführungselemente 34 aufweisen. Die Bandführungselemente 34 sind oberhalb des beiderseitigen Randes des Bandes 2 angeordnet und führen das Band 2 durch den Hohlraum 38 des Schloßgehäuses 4 so, daß das Band 2 glatt auf dem unteren Teil 9 des Schloßgehäuses aufliegt.

Zusätzlich kann das obere Teil 5 des Schloßgehäuses 4 an seinem der Bandschlaufe abgewandten Ende eine Lippe 36 aufweisen, die zwischen den Bandführungselementen 34 angeordnet ist und die vorzugsweise elastisch ausgebildet ist. Der Abstand der Lippe 36 von den durch den unteren Teil 9 des Schloßgehäuses gebildeten Boden ist vorzugsweise etwas kleiner als die Dicke des Bandes 2, so daß der durch das Schloßgehäuse 4 verlaufende Bandabschnitt 3 durch die Lippe 36 leicht geklemmt wird.

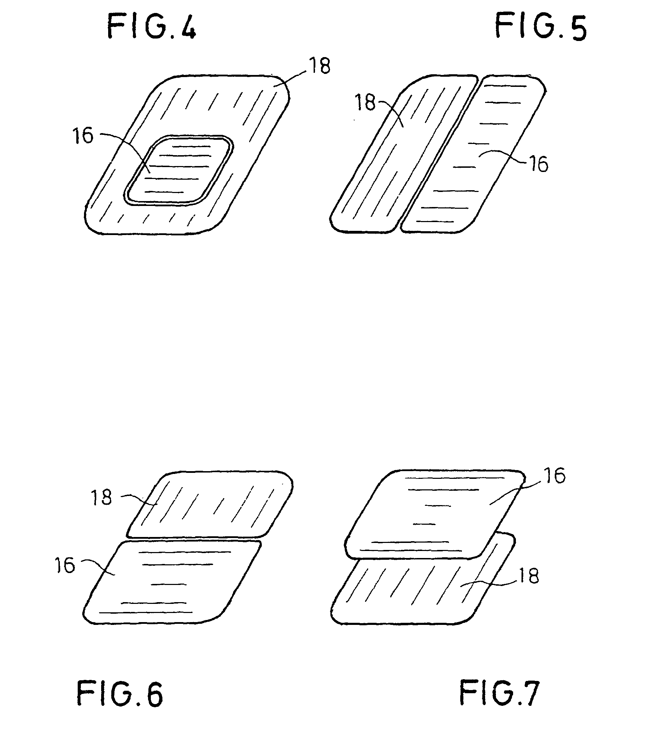

Die Fign. 4 bis 7 zeigen alternative Tastenanordnungen der Tastflächen 16 und 18, wobei Fig. 4 dem in den Fign. 1 und 3 dargestellten Ausführungsbeispiel entspricht.

Fig. 5 zeigt ein Ausführungsbeispiel, bei dem die Tastflächen 16,18 nebeneinander angeordnet ist, während Fig. 6 eine Anordnung zeigt, bei der die Tastflächen 16,18 hintereinander liegen.

Fig. 7 zeigt ein Ausführungsbeispiel, bei dem die erste Tastfläche 16 gegenüber der zweiten Tastfläche 18 höher angeordnet ist. Nach Betätigung der ersten Tastfläche 16 zum Lösen der Bandspannung kann die zweite Tastfläche 18 nach einem voreingestellten Tasthub der ersten Tastfläche 16 ebenfalls betätigt werden, um den Klemmhebel 6 vollständig aus dem Schloßgehäuse zu lösen.

Die Tastflächen 16,18 können unterschiedlich farbig gekennzeichnet sein, um deren Unterscheidung zu erleichtern.

Die Tastfläche können hierzu auch mit einer farbigen Aufsatzkappe versehen sein, die auf die Tastflächen 16,18 befestigt wird.

## Patentansprüche

1. Venenstauvorrichtung (1) für Körperteile, mit einem eine Schlaufe bildenden Band (2), mit einem Schloßgehäuse (4) und einem in dem Schloßgehäuse (4) schwenkbar angeordneten Klemmhebel (6) zum Klemmen des durch das Schloßgehäuse (4) hindurchgeführten Bandabschnittes (3) mit einem ersten Ende (7) des Klemmhebels (6) bei gespannter Schlaufe, wobei ein Ende (8) des Bandes (2) an einem zweiten Ende (10) des Klemmhebels (6) angreift, der aus einem unteren Schenkelteil (20) und einem oberen Schenkelteil (22) gebildet ist, mit Hilfe einer Rastverbindung (12) in das Schloßgehäuse (4) einrastbar ist und mittels einer am Klemmhebel (6) angeordneten zweiten Tastfläche (18) von dem Schloßgehäuse (4) lösbar ist, **dadurch gekennzeichnet, daß** das Band (2) mit dem unteren Schenkelteil (20) des Klemmhebels (6) verbunden ist und daß der Klemmhebel (6) eine erste Tastfläche (16) aufweist, die unmittelbar auf das zweite Ende (10) des Klemmhebels (6) einwirken und bei Betätigung den Klemmhebel (6) ohne Entsperren verschwenken kann.

2. Vorrichtung nach Anspruch 1, **dadurch gekennzeichnet, daß** der obere Schenkelteil (22) elastisch verschwenkbar ist und eine Entsperreinrichtung (14) bildet, die die zweite Tastfläche (18) aufweist.

3. Vorrichtung nach einem der Ansprüche 1 oder 2, **dadurch gekennzeichnet, daß** der Klemmhebel (6) einstückig ist.

4. Vorrichtung nach einem der Ansprüche 1 bis 3, **dadurch gekennzeichnet, daß** der untere Schenkelteil (20) als zweiarmiger Hebel eine quer zum Band verlaufende Schwenkachse aufweist.

5. Vorrichtung nach einem der Ansprüche 1 bis 4, **dadurch gekennzeichnet, daß** der obere Schenkelteil (22) mit dem oberen Teil des Schloßgehäuses (4) über die Rastverbindung (12) verrastet und damit den Klemmhebel (6) im Schloßgehäuse (4) verriegelt.

6. Vorrichtung nach einem der Ansprüche 1 bis 5, **dadurch gekennzeichnet, daß** die erste Tastfläche (16) mit dem unteren Schenkelteil (20) des Klemmhebels (6) starr gekoppelt ist.

7. Vorrichtung nach einem der Ansprüche 1 bis 6, **dadurch gekennzeichnet, daß** die zweite Tastfläche (18) starr mit dem oberen Schenkelteil (22) gekoppelt ist und die Entsperreinrichtung (14) bildet, die bei Betätigung die Rastverbindung (12) löst, wodurch der Klemmhebel (6) aus dem Schloßgehäuse (4) entnehmbar ist und die Schlaufe des Bandes (2) geöffnet wird.

8. Vorrichtung nach einem der Ansprüche 1 bis 7, **dadurch gekennzeichnet, daß** die Rastverbindung (12) aus einem Vorsprung (24) und einer Aussparung (26) bestehen.

9. Vorrichtung nach einem der Ansprüche 1 bis 8, **dadurch gekennzeichnet, daß** der untere Schenkelteil (20) seitlich vorstehende Zapfen (30) aufweist, die in den Zapfen (30) angepaßte Nuten (32) in Seitenwänden (28) des Schloßgehäuses (4) eingreifen und die Schwenkachse des Klemmhebels (6) bilden.

10. Vorrichtung nach einem der Ansprüche 1 bis 9, **dadurch gekennzeichnet, daß** die erste Tastfläche (16) gegenüber der zweiten Tastfläche (18) übersteht, so daß nach Betätigung der ersten Tastfläche (16) um einen vorgegebenen Tasthub auch die zweite Tastfläche (18) zum Lösen des Klemmhebels (6) betätigbar ist.


**Claims**

1. Tourniquet device (1) for extremities comprising a strap (2) forming a loop, with a lock housing (4) and a clamping lever (6) pivotally arranged in the lock housing (4) for clamping the strap portion (3) passed through the lock housing (4) by a first end (7) of the clamping lever (6) when the loop is taut, one end (8) of the strap (2) engaging a second end (10) of the clamping lever (6) which is formed by a lower leg part (20) and an upper leg part (22), may be caught in the lock housing (4) by means of a catch connection (12) and may be detached from the lock housing (4) by means of a second key surface (18) provided on the clamping lever (6), **characterized in that** the strap (2) is connected with the lower leg part (20) of the clamping lever (6) and that the clamping lever (6) has a first key surface (16) that may act directly on the second end (10) of the clamping lever (6) and, when operated, may pivot the clamping lever (6) without unlocking.

2. Device of claim 1, wherein the upper leg part (22) is elastically pivotable and forms an unlocking means (14) having the second key surface (18).

3. Device of one of claims 1 or 2, wherein the clamping lever (6) is integral.

4. Device of one of claims 1 to 3, wherein the lower leg part (20) as a double arm lever has a pivot axis extending transversally to the strap.

5. Device of one of claims 1 to 4, wherein the upper leg part (22) engages the upper part of the lock housing (4) via the catch connection (12), thereby locking the clamping lever (6) in the lock housing (4).

6. Device of one of claims 1 to 5, wherein the first key surface (16) is rigidly coupled to the lower leg part (20) of the clamping lever (6).

7. Device of one of claims 1 to 6, wherein the second key surface (18) is rigidly coupled to the upper leg part (22) and forms the unlocking means (14) that, when operated, disengages the catch connection (12), whereby the clamping lever (6) may be removed from the lock housing (4) and the loop of the strap (2) is opened.

8. Device of one of claims 1 to 7, wherein the catch connection (12) comprises a projection (24) and a recess (26).

9. Device of one of claims 1 to 8, wherein the lower leg part (20) has laterally projecting pins (30) engaging grooves (32) adapted to the pins and formed in the sidewalls (28) of the lock housing (4) and forming the pivot axis of the clamping lever (6).

10. Device of one of claims 1 to 9, wherein the first key surface (16) protrudes beyond the second key surface (18) so that after operating the first key surface (16) by a predetermined key stroke, the second key surface (18) may be operated to disengage the clamping lever (6).


**Revendications**

1. Dispositif (1) d'occlusion veineuse pour des parties du corps, comprenant une bande (2) formant une boucle, un boîtier de serrure (4) et un levier de serrage (6) placé à pivotement dans le boîtier de serrure (4) pour le serrage de la portion de bande (3) passée à travers le boîtier de serrure (4) avec une première extrémité (7) du levier de serrage (6) lorsque la boucle est serrée, une extrémité (8) de la bande (2) étant en prise avec une deuxième extrémité (10) du levier de serrage (6) qui est constitué d'une branche inférieure (20) et d'une branche supérieure (22), s'enclenchant dans le boîtier de serrure (4) à l'aide d'une liaison à encliquetage (12) et pouvant être libérée du boîtier de serrure (4) au moyen d'une deuxième surface de touche (18) située sur le levier de serrage (6),
**caractérisé en ce que**
la bande (2) est reliée à la branche inférieure (20) du levier de serrage (6) et
le levier de serrage (6) comporte une première surface de touche (16) qui agit directement sur la deuxième extrémité (10) du levier de serrage (6) et qui, lors d'une commande, peut faire basculer le levier de serrage (6) sans déverrouillage.

2. Dispositif selon la revendication 1, **caractérisé en ce que** la branche supérieure (22) est apte à basculer de façon élastique et forme un dispositif de déverrouillage (14) qui comporte la deuxième surface de touche (18).

3. Dispositif selon l'une des revendications 1 ou 2, **caractérisé en ce que** le levier de serrage (6) est d'une seule pièce.

4. Dispositif selon l'une des revendications 1 à 3, **caractérisé en ce que** la branche inférieure (20) comporte en tant que levier à deux bras un axe de pivotement s'étendant transversalement à la bande.

5. Dispositif selon l'une des revendications 1 à 4, **caractérisé en ce que** la branche supérieure (22) s'enclenche avec la partie supérieure du boîtier de serrure (4) via la liaison à encliquetage (12) et verrouille en conséquence le levier de serrage (6) dans le boîtier de serrure (4).

6. Dispositif selon l'une des revendications 1 à 5, **caractérisé en ce que** la première surface de touche (16) est couplée rigidement à la branche inférieure (20) du levier de serrage (6).

7. Dispositif selon l'une des revendications 1 à 6, **caractérisé en ce que** la deuxième surface de touche (18) est couplée rigidement à la branche supérieure (22) et forme le dispositif de déverrouillage (14) qui libère, lors d'une commande, la liaison à encliquetage (12), grâce à quoi le levier de serrage (6) peut être retiré du boîtier de serrure (4) et la boucle de la bande (2) est ouverte.

8. Dispositif selon l'une des revendications 1 à 7, **caractérisé en ce que** la liaison à encliquetage (12) est constituée d'une saillie (24) et d'un évidement (26).

9. Dispositif selon l'une des revendications 1 à 8, **caractérisé en ce que** la branche inférieure (20) comporte des ergots (30), faisant saillie latéralement, qui s'engagent dans des gorges (32) adaptées aux ergots (30) et ménagées dans les parois latérales (28) du boîtier de serrure (4) et qui forment l'axe de pivotement du levier de serrage (6).

10. Dispositif selon l'une des revendications 1 à 9, **caractérisé en ce que** la première surface de touche (16) fait saillie par rapport à la deuxième surface de touche (18) de sorte que, après actionnement de la première surface de touche (16) sur une course de touche prédéterminée, la deuxième surface de touche (18) peut également être actionnée pour libérer le levier de serrage (6).

FIG.1

FIG.2

FIG.3

# FIG.4

# FIG.5

# FIG.6

# FIG.7